(19) Europäisches Patentamt
European Patent Office
Office européen des brevets

(11) **EP 4 516 216 A1**

(12) **EUROPEAN PATENT APPLICATION**

(43) Date of publication:
**05.03.2025 Bulletin 2025/10**

(21) Application number: **23193929.9**

(22) Date of filing: **29.08.2023**

(51) International Patent Classification (IPC):
**A61B 5/08** (2006.01)  **A61B 5/113** (2006.01)
**A61B 5/00** (2006.01)

(52) Cooperative Patent Classification (CPC):
**A61B 5/113; A61B 5/0803; A61B 5/0816;
A61B 5/1135; A61B 5/6813; A61B 5/6889;
A61B 5/6893; A61B 5/721; A61B 5/7257;**
A61B 5/6888; A61B 2562/04

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventors:
• **BAZHENOV, Mikhail
  Eindhoven (NL)**
• **FRANCK, Christoph Florian
  Eindhoven (NL)**

(74) Representative: **Philips Intellectual Property &
Standards
High Tech Campus 52
5656 AG Eindhoven (NL)**

(54) **GENERATING A RESPIRATORY SIGNAL**

(57)  A mechanism for monitoring respiration of a subject. A plurality of motion signals are obtained, each motion signal having a different sensitivity to respiration of the subject. The plurality of motion signals are processed using a component analysis technique to produce component signals. One of the component signals is identified as a respiratory signal for the subject.

EP 4 516 216 A1

FIG. 2

**Description**

FIELD OF THE INVENTION

The present invention relates to subject monitoring, and in particular to the monitoring of respiration.

BACKGROUND OF THE INVENTION

Accurate monitoring of respiration of a (medical) subject is important for correctly understanding and assessing the condition of the subject. One known approach for generating a respiratory signal, which changes responsive to at least changes in respiration speech, is to use an electrical impedance. The respiratory signal may then be further processed, for example, to determine one or more respiration parameters such as a respiration rate, respiration (or breath rate) variability, (average) inhalation/exhalation duration and so on.

An alternative method for generating a respiratory signal is to use an accelerometer or other motion detector placed on the chest of the subject to monitor a movement of the chest, and therefore respiration. Such a mechanism can take advantage of low-cost and low complexity circuitry in order to monitor respiration.

As a respiratory signal carries information about the respiration of the (medical) subject, it is preferable to reduce the noise in the respiratory signal. There is therefore a demand for approaches that generate respiratory signals with reduced noise.

SUMMARY OF THE INVENTION

The invention is defined by the claims.

According to examples in accordance with an aspect of the invention, there is provided a computer-implemented method for generating a respiratory signal for a subject.

The computer-implemented method comprises: receiving a plurality of motion signals generated by a respective plurality of motion detectors, wherein each motion signal is configured to change responsive to at least an environmental motion that moves the subject and at least two motion signals have different sensitivities to the respiratory motion of the subject; processing the plurality of motion signals using a component analysis technique to identify two or more component signals; and generating the respiratory signal by processing the two or more component signals to identify which component signal represents the respiratory motion of the subject.

The present disclosure provides a mechanism for extracting a respiratory signal from a plurality of motion signals having different sensitivities to the respiration of the subject. In particular, a component analysis is performed on the motion signals to identify a plurality of component signals. The component signal that represents the respiratory motion of the subject is identified as used as the respiratory signal.

The proposed approach provides a mechanism for defining a respiratory signal that is more resistant to noise, particularly environmental motion, than previously known or described techniques.

In some examples, generating the respiratory signal comprises performing a frequency analysis on each component signal to identify the component signal having the greatest average magnitude at frequency components that match respiratory frequency components. This provides a reliable and efficient mechanism for identifying the component signal most likely to be representative of a respiratory signal.

In some examples, generating the respiratory signal comprises identifying the component signal having a morphology most similar to a respiratory morphology. This provides an alternative mechanism for identifying the component signal most likely to be representative of a respiratory signal that does not require performance of a resource-intensive frequency domain transformation of the component signals.

In some examples, the component analysis technique is a principal component analysis technique.

In other examples, the component analysis technique is an independent component analysis technique.

In some examples, at least one of the motion detectors detects a motion that is distanced from the subject.

The plurality of motion signals may comprise at least three motion signals.

Each motion signal may carry at least two channels. For instance, each motion signal may carry at least three channels. The greater the number of channels, the more information and nuance on respiration that will be carried by the motion signal(s), thereby increasing the accuracy of producing a component signal that appropriately represents a respiratory signal.

In some examples, at least one motion detector detects a motion of a support for the subject. This facilitates the distinction of a motion in an immediate environment to the subject from respiration motion.

In some examples, at least one motion detector detects a motion of a wall, floor or ceiling of an environment in which the subject is located. This facilitates the distinction of a motion in a more general environment to the subject from respiration motion.

The environment may be a vehicle. This embodiment recognizes that there is a particular problem with distinguishing respiratory motion from vehicular motion (e.g., during transport of a (medical) subject, such as on an ambulance). This embodiment thereby improves the ability to distinguish between vehicular motion and respiratory motion.

Each motion detector may be an accelerometer, such that each motion signal comprises accelerometer data.

There is also provided a computer program comprising code means for implementing any herein disclosed method when said program is run on a processing system. There is provided a computer-readable data carrier having stored thereon the computer program and/or code means previously described. Preferably, the computer-readable data carrier is non-transitory.

There is also provided a processing system for generating a respiratory signal for a subject, wherein the processing system is configured to: receive a plurality of motion signals generated by a respective plurality of motion detectors, wherein each motion signal is configured to change responsive to at least an environmental motion that moves the subject and at least two motion signals have different sensitivities to the respiratory motion of the subject; process the plurality of motion signals using a component analysis technique to identify two or more component signals; and generate the respiratory signal by processing the two or more component signals to identify which component signal represents the respiratory motion of the subject.

There is also provided a respiratory monitoring system comprising the processing system; and the plurality of motion detectors.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates a respiratory monitoring system; and
Fig. 2 is a flowchart illustrating a proposed method.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for monitoring respiration of a subject. A plurality of motion signals are obtained, each motion signal having a different sensitivity to respiration of the subject. The plurality of motion signals are processed using a component analysis technique to produce component signals. One of the component signals is identified as a respiratory signal for the subject.

Proposed approaches rely upon the recognition that a motion signal that responds to respiration of a subject is also subject to motion noise (e.g., due to a movement of an environment in which the subject is located). In the motion signal, respiration motion and environmental motion are distinct/uncorrelated with respect to one another. It has been recognized that component analysis allows the separation of these two components in component signals.

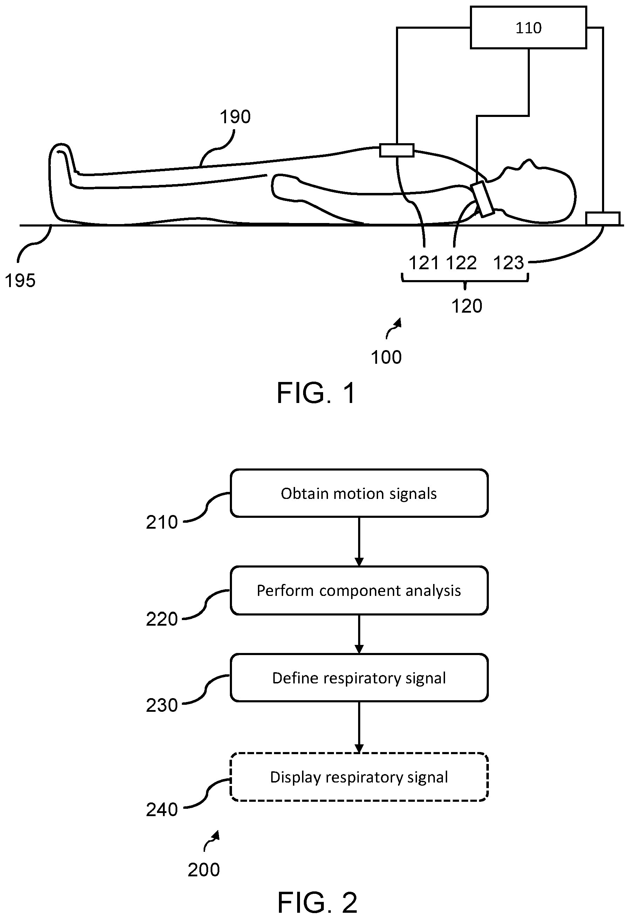

Figure 1 illustrates a respiratory monitoring system 100 for use with proposed embodiments, for improved contextual understanding. The respiratory monitoring system 100 is configured for monitoring a respiration of a (medical) subject 190. In the illustrated example, the subject 190 is on a support 195.

The respiratory monitoring system 100 comprises a processing system 110 (itself a proposed embodiment) and a plurality of motion detectors 120. The plurality of motion detectors comprises more than one motion detector, e.g., at least a first motion detector 121 and a second motion detector 122. The plurality of motion detectors may comprise one or more further motion detectors, e.g., a third motion detector 123.

Each motion detector 121, 122, 123 is configured to generate a motion signal responsive to detected motion. Each motion signal may comprise, for instance, an iteratively updated set of motion values, wherein a change or difference between motion values is indicative of a motion. Thus, each motion signal may comprise a discretized or sample of motion of the subject.

In the context of the present disclosure, a motion signal is a signal generated by a single motion detector. A single motion signal may, for instance, comprise one or more channels (e.g., each channel representing a motion in a different direction).

The motion signal generated by each motion detector is responsive to at least an environment in which the subject 190 is located. This may comprise, for instance, a motion of a support 195 upon which the subject 190 is positioned (e.g., caused by a vibratory element for performing vibration treatment or the like) and/or a motion of a room or other enclosed environment (e.g., ambulance compartment) in which the subject is located.

At least one motion signal is responsive to respiration of the subject 190. Thus, at least one motion signal is responsive to a motion of the subject's chest or thorax, e.g., indicative of a movement of the diaphragm.

Suitable examples of motion detectors, and corresponding motion signals, are known in the art, and include accelerometers, gyrometers, electromagnetic sensors; remote motion sensors (e.g., ultrasound-based sensors, time-of-flight based sensors, camera based sensors and so on).

The motion signals are passed to the processing system 110, which processes the motion signals to generate or determine a respiratory signal.

The motion detectors and processing system may communicate by any suitable wired or wireless protocol, e.g., directly, via a LAN connection or via the internet. In some examples, the motion detectors may store the motion signals in a database or memory, from which they are retrieved by the processing system.

Suitable wireless communication protocols that may be used for communication include an infrared link, Zigbee, Bluetooth, a wireless local area network protocol such as in accordance with the IEEE 802.11 standards, a 2G, 3G or 4G telecommunication protocol, an internet-based protocol or format and so on. Other formats will be readily apparent to the person skilled in the art.

The present disclosure proposes to process at least two motion signals that are responsive (i.e., change in value) to an environmental motion. At least two of the motion signals have different sensitivities to the respiration motion of the subject. In particular, at least two of the motion signals have linearly independent sensitivities to the respiration motion of the subject.

This implies that at least one of the motion signals has a non-zero sensitivity to the respiration motion of the subject.

In general, a motion signal x produced by a motion detector in the context of the proposed use-case scenario can be assumed to be a linear combination of two, uncorrelated (source) signals $s_1$ and $s_2$, where $s_1$ is a respiratory signal (e.g., responsive to respiration) and $s_2$ is a noise signal (e.g., responsive to environmental motion). More particularly, an n-th motion signal $x_n$ can be mathematically expressed as:

$$x_n = a_n . s_1 + b_n + s_2 \qquad\qquad (1)$$

where $a_n$ is a first coefficient for the n-th motion signal and $b_n$ is a second coefficient for the n-th motion signal. The first and second coefficients can be labelled mixing coefficients.

The present invention assumes that, for at least an n-th motion signal and an n+1-th motion signal, the vectors $(a_n, a_{n+1})$ and $(b_n, b_{n+1})$ are linearly independent. This facilitates separation of the respiratory signal $s_1$ and the motion signal $s_2$.

It is proposed to perform a component analysis technique on the plurality of motion signals. Thus, the plurality of motion signals are input to a component analysis technique. The output of the component analysis technique is a plurality of component signals. Generally, the number of component signals produced is up to the number of the plurality of motion signals that are processed.

The component analysis technique thereby acts to separate the contributing uncorrelated signals to a motion signal from one another. Example component analysis techniques include (functional) principle component analysis techniques and (functional) independent component analysis techniques.

The principle and performance of component analysis techniques are known in the art. The present disclosure proposes the application of such techniques for the identification of a component signal of more than one motion signals as a respiration signal.

It will be apparent that one of the component signals will represent respiration of the subject and at least one other will represent motion noise, e.g., caused by environmental motion noise. This understanding holds true even if only one of the motion signals input to the component analysis technique is responsive to respiration. Thus, it is possible to select one of the component signals to act as the respiratory signal for the subject. This produces a noise-filtered respiratory signal.

Figure 2 is a flowchart illustrating a computer-implemented method 200 for generating a respiratory signal for the subject. The method 200 may be carried out by a processing system, e.g., the processing system previously described.

The method 200 comprises a step 210 of receiving a plurality of motion signals generated by a respective plurality of motion detectors. As previously mentioned, each motion signal is configured to change responsive to at least an environmental motion that moves the subject and at least two motion signals have different sensitivities to the respiratory motion of the subject.

The method 200 also comprises a step 220 of processing the plurality of motion signals using a component analysis technique to identify two or more component signals. The component analysis technique may be a principal component analysis technique or an independent component analysis technique. As another examples, the component analysis technique may be an independent component analysis technique.

The method 200 also comprises a step 230 of generating the respiratory signal by processing the two or more component signals to identify which component signal represents the respiratory motion of the subject.

A first approach approach for performing step 230 comprises performing a frequency analysis on each component signal to identify the component signal having the greatest average magnitude at frequency components that match respiratory frequency components.

In this first approach, each component signal undergoes a temporal frequency transform, e.g., using a Fourier-based transform (such as an FFT or STFT), to produce a frequency spectrum - i.e., a frequency domain representation of the component signal. The frequency spectrum may be processed to identify the frequency spectrum that contains the greatest magnitude at respiration frequencies, which can therefore be attributed to respiration motion of the subject (and not environmental motion).

Respiration frequencies can be readily identified from known respiration rates. For instance, respiration of a human will usually be (taking into account potential bradypnea and increased respiration rate for infants) in the range of from 6 breaths a minute to 60 breaths a minute. Thus, respiration frequencies may be frequencies in the region of from 0.1 Hz to 1 Hz. Other suitable ranges for respiration frequencies will be apparent to the skilled person, e.g., to take into account specific expected respiration rates or exaggerated respiration rates. For instance, to ensure an outlying respiration rate is captured, the respiration frequencies may be in the range from 0.02 Hz to 2 Hz.

In a second approach, generating the respiratory signal comprises identifying the component signal having a morphology most similar to a respiratory morphology.

This second approach recognizes that the shape of a motion signal that is responsive to only respiratory motion will have a predictable or known shape or morphology (e.g., as lung/diaphragm movement follows a known pattern). By identifying the component signal having a morphology most similar to a respiratory morphology, the component signal most likely to represent the respiration of the subject can be immediately identified.

As a basic example, a respiratory morphology may have been generated by using a motion sensor to monitor respiration motion of a sample or example subject in a closed environment (e.g., a near-noiseless environment with regard to motion). This respiratory morphology may be stored in a database for later retrieval and/or reference. In some examples, the respiratory morphology is a population-averaged respiratory morphology, e.g., an average of respiratory morphologies captured using the previously described process.

The method 200 may optionally further comprise a step 240 of displaying the identified component signal, e.g., as a user interface or display. Thus, step 240 may comprise controlling a user interface to provide a visual representation of the identified component signal.

In some examples, step 230 further comprises performing additional processing (e.g., further noise filtering) on the identified component signal in defining the respiratory signal. For instance, the identified component signal may be filtered by a low-pass filter, e.g., having a cut-off frequency of no more than 5 Hz (e.g., to ensure the component signal comprises only respiratory-related information).

The method 200 may be adapted to perform one or more other functions or processes with the respiratory signal (the identified component signal).

In some examples, the method comprises storing the respiratory signal in a database for later use.

In some examples, the method comprises comparing the (primary frequency of the) respiratory signal to one or more alarm threshold to determine whether or not to generate an alarm (e.g., if a detected respiration frequency breaches some predetermined threshold).

In some examples, the method comprises passing the respiratory signal to another processing system for further processing any analysis.

A wide variety of other uses for a respiratory signal will be readily apparent to the skilled person.

As previously explained, the plurality of motion detectors that generate the plurality of motion signals have different sensitivities to one another. In particular, it is preferable that the mixing coefficients of the motion signals are independent from one another.

The different sensitivities of the motion detectors can be achieved by positioning the motion detectors at different locations and/or different orientations with respect to one another. Preferably, the motion detectors are positioned at different locations.

Suitable example locations for motion detectors are hereafter described. The location of a motion detector is the location at which motion is detected by the motion detector. For some types of motion detector, such as an accelerometer, the location will be the location at which the motion detector is positioned. For other types of motion detector, such as remote motion detectors, this will be a location remotely monitored by the motion detector. An alternative label for the location of a motion detector is therefore a "motion monitoring location".

At least one motion detector will be configured to detect or be responsive to a respiratory motion of the subject. In particular, at least one motion detector may be configured to detect or be responsive to a motion of the chest or thorax. As an example, at least one motion detector may be an accelerometer positioned on the chest or thorax of the subject.

In some examples, more than one motion detector is configured to detect or respond to a same ambient torso motion of the subject, e.g., a motion of the torso that is not attributable to respiration and/or a limb-specific motion (such as arm motion). This may, for instance, be an ambulant motion of the subject. For instance, the at least two motion detectors may comprise a motion detector be configured to detect or be responsive to a motion of the chest or thorax and a motion detector configured to detect or be responsive to a motion of medical subject that is non-responsive (or negligibly responsive) to respiration, e.g., the shoulder or side of the stomach.

In preferred examples, more than one motion detector is configured to detect or be responsive to respiratory motion of the subject. In such circumstances, it is preferred that each motion detector picks up the respiration motion with different amplitudes and/or directions. As an example, one motion detector may detect a motion on an front part of the chest (e.g., on the breastbone) and another may detect a motion on a side or back of the torso. For instance, the plurality of motion detectors may comprise a first accelerometer positioned on front part of the torso and a second accelerometer positioned on a side or back of the torso.

In at least one example, at least one of the motion detectors detects (only) a motion that is distanced from the subject. This ensures that at least one motion signal is directly responsive to an environmental noise in which the subject is located.

In some examples, at least one motion detector detects a motion of a support for the subject (e.g., and not a motion of the subject themselves). This provides a motion signal that carries environmental noise in the near vicinity of the subject. Examples of supports include beds, gurneys, couches and so on. As an example, at least one motion detector may be an accelerometer positioned on the support for the subject, e.g., coupled to an element (e.g., a patient monitor) attached (and preferably rigidly attached) to the support.

In some examples, at least one motion detector detects a motion of a wall, floor or ceiling of an environment in which the subject is located (e.g., and not a motion of the subject themselves). This provides a motion signal that carries environmental motion noise in the environment of the subject. As an example, at least one motion detector may be an accelerometer positioned on the motion of the wall, floor or ceiling, e.g., coupled to an element (e.g., a patient monitor, cabinet or the like) rigidly attached to the wall, floor or ceiling of the vehicle.

The environment may be a vehicle, such that the motion detector detects a motion of the vehicle. This advantageously produces a motion signal that contains environmental noise due to a vehicular motion, allowing for distinguishing of vehicular motion from respiratory motion.

As one working example, the plurality of motion sensors may comprise a first accelerometer attached to the torso of the subject and a second accelerometer rigidly attached to a wall of a vehicle.

As another working example, the plurality of motion sensors may comprise a first accelerometer attached to the torso of the subject and a second accelerometer rigidly attached to a support of the subject (e.g., a bedframe or gurney supporting the subject).

As another working example, the plurality of motion sensors may comprise a first accelerometer attached to the torso of the subject and a second accelerometer rigidly attached to the subject in a location for which no respiration motion is present or that any detected respiration is uncorrelated with the respiration detected by the first accelerometer. For instance, the second accelerometer may be positioned on a shoulder of the subject.

It will be appreciated that any combination of the above-identified locations may be used. In particular, the plurality of motion signals may comprise more than two motion signals from a corresponding number of motion detectors located at any combination of the aforementioned locations.

The skilled person would be readily capable of developing a processing system for carrying out any herein described method. Thus, each step of the flow chart may represent a different action performed by a processing system, and may be performed by a respective module of the processing system.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

There is, of course, also proposed a respiratory monitoring system comprising the processing system and the plurality of motion detectors. Each motion detector may be appropriately configured to one of the motion monitoring locations previously described.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method. Thus, there is provided a computer-readable data carrier having stored thereon the computer program previously described.

In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. The mere fact that certain measures are recited in mutually different dependent claims does not indicate that a combination of these measures cannot be used to advantage.

In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

A single processor or other unit may fulfill the functions of several items recited in the claims. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. A computer-implemented method (200) for generating a respiratory signal for a subject (190), the computer-implemented method comprising:

   receiving (210) a plurality of motion signals generated by a respective plurality (120) of motion detectors (121, 122, 123), wherein each motion signal is configured to change responsive to at least an environmental motion that moves the subject and at least two motion signals have different sensitivities to the respiratory motion of the subject;
   processing (220) the plurality of motion signals using a component analysis technique to identify two or more component signals; and
   generating (230) the respiratory signal by processing the two or more component signals to identify which component signal represents the respiratory motion of the subject.

2. The computer-implemented method of claim 1, wherein generating the respiratory signal comprises performing a frequency analysis on each component signal to identify the component signal having the greatest average magnitude at frequency components that match respiratory frequency components.

3. The computer-implemented method of claim 1, wherein generating the respiratory signal comprises identifying the component signal having a morphology most similar to a respiratory morphology.

4. The computer-implemented method of any of claims 1 to 3, wherein the component analysis technique is a principal component analysis technique.

5. The computer-implemented method of any of claims 1 to 3, wherein the component analysis technique is an independent component analysis technique.

6. The computer-implemented method of any of claims 1 to 5, wherein at least one of the motion detectors (123) detects a motion that is distanced from the subject.

7. The computer-implemented method of any of claims 1 to 6, wherein the plurality of motion signals comprises at least three motion signals.

8. The computer-implemented method of any of claims 1 to 7, wherein each motion signal carries at least two channels, and preferably at least three channels.

9. The computer-implemented method of any of claims 1 to 8, wherein at least one motion detector (123) detects a motion of a support (195) for the subject.

10. The computer-implemented method of any of claims 1 to 9, wherein at least one motion detector detects a motion of a wall, floor or ceiling of an environment in which the subject is located.

11. The computer-implemented method of claim 10, wherein the environment is a vehicle.

12. The computer-implemented method of any of claims 1 to 11, wherein each motion detector is an accelerometer, such that each motion signal comprises accelerometer data.

13. A computer program comprising code means for implementing the method of any of claims 1 to 12 when said program is run on a processing system

14. A processing system (110) for generating a respiratory signal for a subject (190), wherein the processing system is configured to:

receive (210) a plurality of motion signals generated by a respective plurality (120) of motion detectors (121, 122, 123), wherein each motion signal is configured to change responsive to at least an environmental motion that moves the subject and at least two motion signals have different sensitivities to the respiratory motion of the subject;
process (220) the plurality of motion signals using a component analysis technique to identify two or more component signals; and
generate (230) the respiratory signal by processing the two or more component signals to identify which component signal represents the respiratory motion of the subject.

15. A respiratory monitoring system (100) comprising:

the processing system of any claim 14; and
the plurality (120) of motion detectors (121, 122, 123).

FIG. 1

FIG. 2

Europäisches
Patentamt
European
Patent Office
Office européen
des brevets

# EUROPEAN SEARCH REPORT

Application Number

EP 23 19 3929

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2023/089523 A1 (E NOVIA S P A [IT]) 25 May 2023 (2023-05-25) * page 3, line 20 - line 29; figures 1, 9 * * page 8, line 6 - line 12; figure 9 * * page 8, line 26 - page 9, line 2 * * page 15, line 8 - line 17 * * page 16, line 3 - line 15 * * page 5, line 22 - line 26 * * claim 3 * * page 7, line 18 - line 20 * | 1-15 | INV. A61B5/08 A61B5/113 A61B5/00 |
| X | WARNECKE JOANA M ET AL: "Noise Reduction for Efficient In-Vehicle Respiration Monitoring with Accelerometers", 2019 41ST ANNUAL INTERNATIONAL CONFERENCE OF THE IEEE ENGINEERING IN MEDICINE AND BIOLOGY SOCIETY (EMBC), IEEE, 23 July 2019 (2019-07-23), pages 3257-3261, XP033625560, DOI: 10.1109/EMBC.2019.8857654 [retrieved on 2019-10-02] * section: B. Data processing; figures 1,2 * | 1-15 | |
| X | CESAREO AMBRA ET AL: "A Wearable Device for Breathing Frequency Monitoring: A Pilot Study on Patients with Muscular Dystrophy", SENSORS, vol. 20, no. 18, 18 September 2020 (2020-09-18), page 5346, XP093099148, DOI: 10.3390/s20185346 * sections: 2.1 Device description, 2.2. Analysis Algorithm * | 1-15 | |

TECHNICAL FIELDS SEARCHED (IPC)

A61B

-/--

The present search report has been drawn up for all claims

| Place of search | Date of completion of the search | Examiner |
|---|---|---|
| The Hague | 7 December 2023 | Papanikolaou, V |

EPO FORM 1503 03.82 (P04C01)

page 1 of 2

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ANGELUCCI ALESSANDRA ET AL: "An IMU-Based Wearable System for Respiratory Rate Estimation in Static and Dynamic Conditions", CARDIOVASCULAR ENGINEERING AND TECHNOLOGY, vol. 14, no. 3, 27 February 2023 (2023-02-27), pages 351-363, XP093099162, Cham ISSN: 1869-408X, DOI: 10.1007/s13239-023-00657-3 * page 355, col. 1, par. 3, Fig. 2 * | 1-15 | |

-----

TECHNICAL FIELDS
SEARCHED        (IPC)

The present search report has been drawn up for all claims

| Place of search | Date of completion of the search | Examiner |
|---|---|---|
| The Hague | 7 December 2023 | Papanikolaou, V |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 23 19 3929

This annex lists the patent family members relating to the patent documents cited in the above-mentioned European search report.
The members are as contained in the European Patent Office EDP file on
The European Patent Office is in no way liable for these particulars which are merely given for the purpose of information.

07-12-2023

| Patent document cited in search report | Publication date | Patent family member(s) | Publication date |
|---|---|---|---|
| WO 2023089523 A1 | 25-05-2023 | NONE | |

For more details about this annex : see Official Journal of the European Patent Office, No. 12/82